# EUROPEAN PATENT APPLICATION

(11) **EP 2 578 687 A1**
(43) Date of publication of application: **10.04.2013**
(21) Application number: 12191735.5
(22) Date of filing: 03.12.2008
(51) Int. Cl.: C12N 15/82

(54) **Seed-preferred gene promotors from the castor plant**

(30) Priority: 04.12.2007 US 992273 P
(62) Divisional of application: 08857967.7
(71) Applicant: DOW GLOBAL TECHNOLOGIES INC., Midland MI 48674 (US)
(72) Inventor: Roessler, Paul G., San Diego, CA 92127 (US); Rasochova, Lada, Del Mar, CA 92014 (US); Lee, Vincent D., San Diego, CA 92129 (US)
(74) Representative: Ward, Siobhan

(57) **Abstract**

A nucleotide sequence comprising a nucleotide sequence having at least about 90% homology to the sequence of SEQ ID NO:9 or SEQ ID NO:10. Nucleotide sequences of interest operatively linked to nucleotide sequence having at least about 90% homology to the sequence of SEQ ID NO:9 or SEQ ID NO:10 are disclosed. Vectors, methods of regulating target expression, methods of providing a cells, plants, and seeds comprising the nucleotide sequence are also disclosed.

SEQUENCE LISTING

<110>  Roessler, Paul  G.
       Rasochova, Lada

<120>  SEED-PREFERRED GENE PROMOTERS FROM THE CASTOR PLANT

<130>  2971-8224

<160>  10

<170>  PatentIn version 3.3

<210>  1
<211>  22
<212>  DNA
<213>  Artificial

<220>
<223>  PCR Primer

<400>  1
gtaatacgac tcactatagg gc                                              22


<210>  2
<211>  28
<212>  DNA
<213>  Artificial

<220>
<223>  PCR Primer

<400>  2
gagagcagcg aagaaggctg aaccatag                                        28


<210>  3
<211>  19
<212>  DNA
<213>  Artificial

<220>
<223>  PCR Primer

<400>  3
actatagggc acgcgtggt                                                  19


<210>  4
<211>  28
<212>  DNA
<213>  Artificial

<220>
<223>  PCR Primer

<400>  4
gagagccatg gaagagaaca agtaggaa                                        28


<210>  5
<211>  22
<212>  DNA
<213>  Artificial

<220>
<223>  PCR Primer

<400>  5
gtaatacgac tcactatagg gc                                                22

<210>  6
<211>  30
<212>  DNA
<213>  Artificial

<220>
<223>  PCR Primer

<400>  6
ataggcttgc agaatcagag cttctggtta                                        30

<210>  7
<211>  19
<212>  DNA
<213>  Artificial

<220>
<223>  PCR Primer

<400>  7
actatagggc acgcgtggt                                                    19

<210>  8
<211>  30
<212>  DNA
<213>  Artificial

<220>
<223>  PCR Primer

<400>  8
ggtgactaac aaccggtgat tgttgatgct                                        30

<210>  9
<211>  1124
<212>  DNA
<213>  Ricinus communis

<400>  9
aactactgcc gctgcgtcaa ttaattctca tagtcctgaa atgacagagg atcaatactt      60

gcatttacta atcttaattt ctttctaagc catgcaccaa ataaattcaa agctaaacaa     120

aagaactaca tatttttttt aaaaagagaa aaaatattag aggataagga ggtggttaga     180

ttgaagaaat cttatgaagg accaacagtg atacttacta taaacagaac aaagattatg     240

agggtttaac acccattaat gttgaagctt agacagactt ggtacctgaa gggacattta     300

ggggacgatg gtcattaatt gccctccagg ctggtcttta tttgttggca tctatgtggt     360

tgtaactctg gttacaacta gctagggtta tttgagcaag tgtatatctt ttcgaattaa     420

taagagtttt aagtgtatat atataggatt ggtgttctta aagtattact agtaaagtat     480

catacagttt gaattatagt atgagatttg tttaagtaaa tattatagac aacaaaatat     540

gtttaagtaa agtatattaa attatcaatc tgtaaatata acgaaataga tcaaacattg     600

actccactgg atagatcatc ttctgtatgt gtgatttaaa caattgggca tggagatgca     660

aaagagcgtt atattaaatg caaaagaaga aaagaaatct aacatttgtc cctaacctct     720

ctttacttgg acgttaaaga tcaaacatcg agttaaagta ctaaatcaac catagttatg 780

cctacatgac acttcaattg ctaaatttca accatagtta ttagacactt gatcactttt 840

tggtgttgtg gtaatggttt ttgcaatcca atgttgtctt cttctttttt ctttttcccc 900

attcacaaac ttttgtattc ttctatggag ttttgataca cctgccctat ctgactttat 960

taggtgtaag aagttaagat gaagtagcca tgcaaatcaa agagagtgac caaagaaacc 1020

ttacgatcat ctttcttttg catctccttc ttcctctata aataccaacc ccttccattc 1080

gccacttcca tcccagaacc acctatcccc tcctctctgc cact 1124

<210> 10
<211> 528
<212> DNA
<213> Ricinus communis

<400> 10
acttggaccc ttgatgccgc attaatttat tacattagtg aggagaagtt ttaaacgtgg 60

agatcatatg tttgatacta ctgtttttgc caaaagttct tttattgtat aaagagaatt 120

ggtgatatag tgtggtaatg aatttgatga atcaacacat agttgtagcc agaaagaacc 180

ctcgaatcga cgccaacttt gcttgtcgtt gtgcagtttc ttggagttca ttttatcaca 240

attcacagct cctttttgaa caaactcgaa atcaaacaaa aacaaacgtg tcctaaaatg 300

gaaccacctc gtttgcatgc aacccttgca tggcaacctc ctcaacacgt gcctcttctc 360

tcttcaacac gtgccaatcc tcttgcgacc tatcttccac tctctctcta taaatcacac 420

cgtcccgtcc cccaaaatct tgcatcgctc ccttactccg caaaaaagca atcatctcca 480

tatattttat tatatattaa ccagaagctc tgattctgca agcctatt 528

## Description

### PRIORITY CLAIM

This application claims the benefit of the filing date of United States Provisional Patent Application Serial No. 60/992,273, filed December 4, 2007, for "Seed-Preferred Gene Promoters From the Castor Plant."

### STATEMENT REGARDING FEDERALLY SPONSORED RESEARCH OR DEVELOPMENT

Work described herein was supported in part by an award from the Department of Energy; award number DE-FC07-01ID14213. The United States Government may have certain rights in the invention.

### TECHNICAL FIELD

The present invention relates to nucleotide sequence capable of driving the expression of other nucleotide sequences.

### BACKGROUND

An important consideration for the production of recombinant plants is the use of a gene promoter that exhibits appropriate activity in driving transgene expression. Promoters can control not only the level of gene expression, but also the timing and tissue specificity of expression.

There is interest in developing industrial oilseed crops that can be used to produce fatty acids having specific industrial utility. The castor plant (*Ricinus communis*) is of particular interest in this regard because of its long history of cultivation for castor oil. Therefore, promoters from castor for use in the production of transgenic varieties of castor and other plants would be an advancement in the art.

### DISCLOSURE OF THE INVENTION

Example embodiments of the present invention include nucleotides sequences, vectors, cells, plants, and/or seeds comprising an isolated nucleotide sequence comprising a nucleotide sequence having at least about 90% homology to the sequence of SEQ ID NO:9 or SEQ ID NO:10.

Additional example embodiments of the present invention include nucleotides sequences, vectors, cells, plants, and/or seeds comprising a nucleotide sequence of interest operably linked to an isolated nucleotide sequence comprising a nucleotide sequence having at least about 90% homology to the sequence of SEQ ID NO:9 or SEQID NO:10.

A further example embodiment of the present invention comprises a method of promoting the expression of a molecule encoded by a nucleotide sequence of interest, the method comprising: operatively linking the isolated nucleotide sequence of claim 1 to the nucleotide sequence of interest.

An additional example embodiment of the present invention comprises a method of producing a molecule encoded by a nucleotide sequence of interest in a cell, the method comprising: operatively linking the isolated nucleotide sequence of claim 1 to the nucleotide sequence of interest; providing the operatively linked nucleotide sequences to the cell; and expressing the nucleotide sequence of interest.

An additional example embodiment of the present invention comprises a method of modulating the expression of a target in a cell, the method comprising: providing a nucleotide sequence of interest, wherein the nucleotide sequence encodes an antisense oligonucleotide or an siRNA that is capable of modulating the expression of the target in a cell; operatively linking the isolated nucleotide sequence of claim 1 to the nucleotide sequence of interest; providing the operatively linked nucleotide sequences to the cell; and expressing the nucleotide sequence of interest.

### BRIEF DESCRIPTION OF THE DRAWINGS

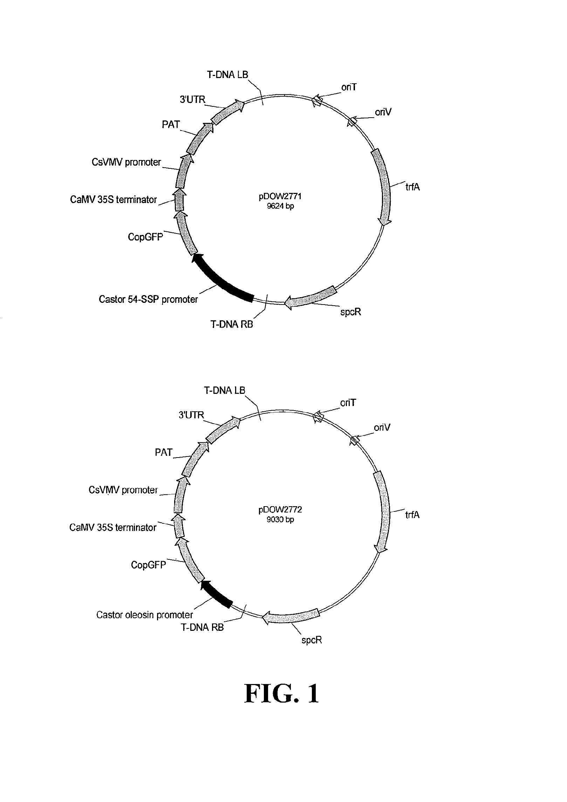

FIG. 1 graphically depicts plant binary expression vectors for testing the castor oleosin and 54-SSP promoters in whole plants.

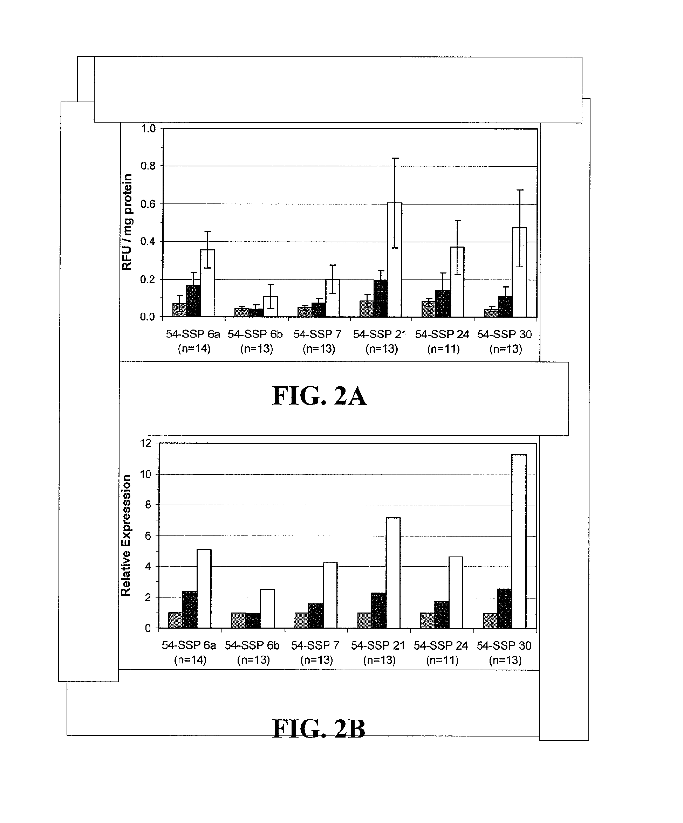

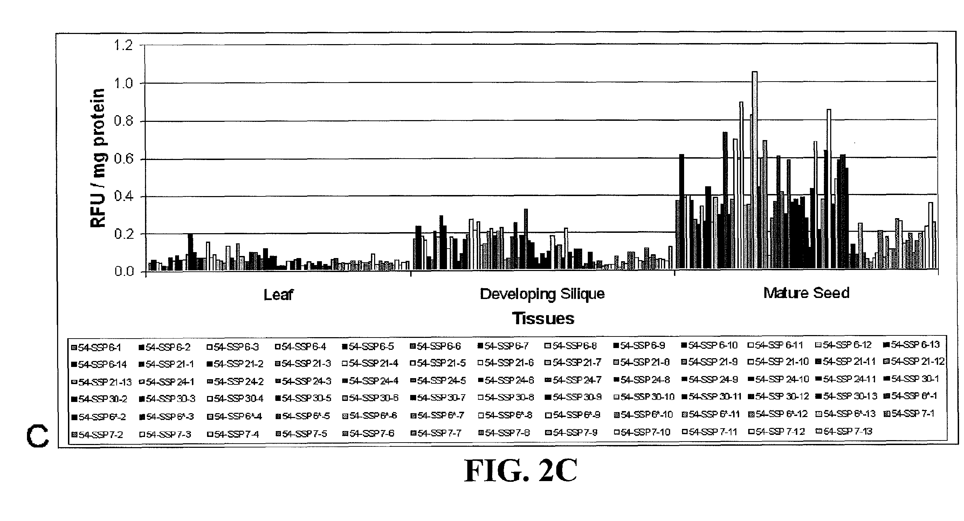

FIGs. 2A, 2B, and 2C are graphical representations of the expression of CopGFP in extracts from various tissues isolated from T2 *Arabidopsis thaliana* plants transformed with pDOW2771 (54-SSP promoter). FIG. 2A depicts mean CopGFP expression results (+/- standard deviations) for T2 plants generated from six different T1 lines. For each T2 line the left bar corresponds to leaf expression, the middle bar to developing silique expression, and the right bar to mature seed expression. FIG. 2B depicts CopGFP expression in various tissues relative to GFP expression in leaves. For each T2 line, the left bar corresponds to leaf:leaf expression, the middle bar to developing silique:leaf expression, and the right bar to mature seed:leaf expression. FIG. 2C depicts CopGFP expression in individual T2 lines. RFU = Relative Fluorescence Units; n = the number of T2 plants examined from each T1 line.

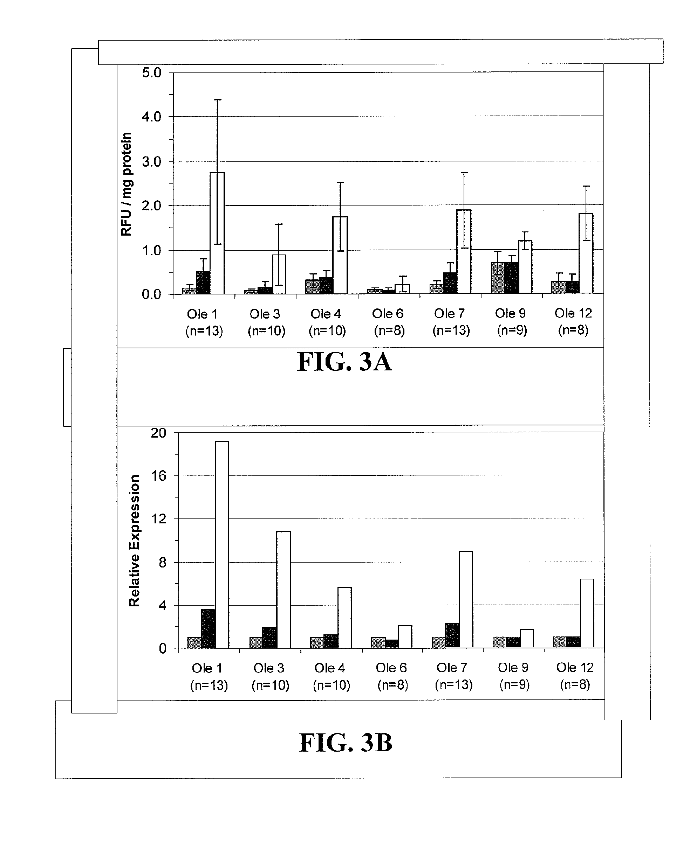

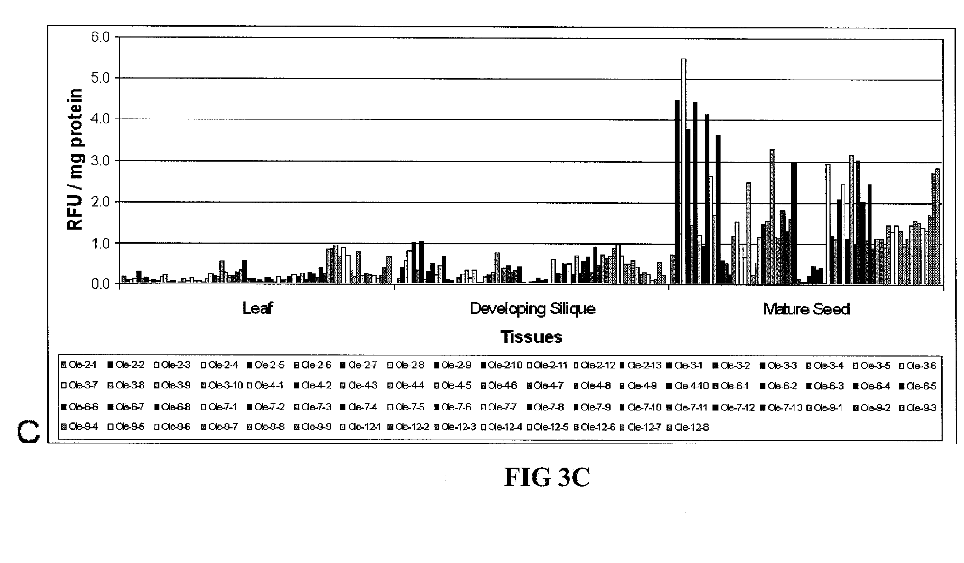

FIGs. 3A, 3B, and 3C are graphical representations of the expression of CopGFP in extracts from various tissues isolated from T2 *Arabidopsis thaliana* plants transformed with pDOW2772 (oleosin promoter). FIG. 3A depicts mean CopGFP expression results (+/- standard deviations) for T2 plants generated from seven different T1 lines For each T2 line, the left bar corresponds to leaf expression, the middle bar to developing silique expression, and the right bar to mature seed expression. FIG. 3B depicts CopGFP expression in various tissues relative to GFP expression in leaves. For each T2 line, the left bar corresponds to leaf:leaf expression, the middle bar to developing silique:leaf expression, and the right bar to mature seed:leaf expression. FIG. 3C depicts CopGFP expression in individual T2 lines. RFU = Relative Fluorescence Units; n = the number of T2 plants examined from each T1 line.

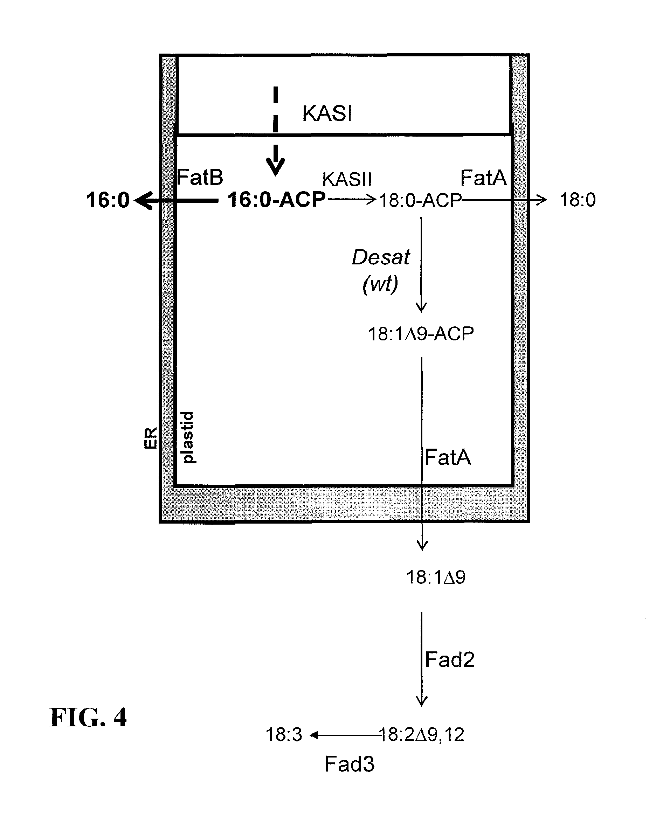

FIG. 4 is a schematic diagram of fatty acid production in *Arabidopsis.*

### MODE(S) FOR CARRYING OUT THE INVENTION

The present invention relates to nucleotide sequences capable of driving the expression of other nucleotide sequences. One aspect of the present invention provides an isolated nucleotide sequence that comprises a nucleotide sequence having at least about 60% identity, at least about 70% identity, at least about 80% identity, at least about 90% identity, or at least about 95% identity to a nucleotide sequence selected from SEQ ID NO:9 and SEQ ID NO:10. In further aspects of the present invention, such a sequence is capable of acting as a promoter.

Further aspects of the present invention provide vectors comprising an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10.

Further aspects of the present invention provide cells comprising an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10.

Further aspects of the present invention provide cells comprising a vector comprising an isolated nucleotide sequence having at least about 60% identity, at least about 70% identity, at least about 80% identity, at least about 90% identity, or at least about 95% identity to a nucleotide sequence selected from SEQ ID NO:9 and SEQ **ID** NO: 10. As will be apparent to one of ordinary skill in the art, a cell can be any kind of cell capable of harboring a nucleotide sequence and/or a vector. Example of cells useful according to the present invention include, but are not limited to, eukaryotic cells, prokaryotic cells, animal cells, plant cells, bacterial cells, germ-line cells, seed cells, *Arabidopsis sp.* cells, sunflower cells, cotton cells, rapeseed cells, maize cells, palm cells, tobacco cells, peanut cells, soybean cells, and *Ricinus sp.* cells.

Further aspects of the present invention provide plants comprising an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10. Examples of plants useful according to the present invention include, but are not limited to, sunflower, cotton, rapeseed, maize, palm, tobacco, peanut, soybean, *Arabidopsis sp.,* and *Ricinus sp.*

Further aspects of the present invention provide seeds comprising an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10. Examples of seeds useful according to the present invention include, but are not limited to, seeds from sunflower, cotton, rapeseed, maize, palm, tobacco, peanut, soybean, *Arabidopsis sp.,* and *Ricinus sp.*

Further aspects of the present invention provide a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO:10.

Further aspects of the present invention provide vectors comprising a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10.

Further aspects of the present invention provide cells comprising a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10.

Further aspects of the present invention provide cells comprising a vector comprising a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO:10. As will be apparent to one of ordinary skill in the art, a cell can be any kind of cell capable of harboring a nucleotide sequence. Example of cells useful according to the present invention include, but are not limited to, eukaryotic cells, prokaryotic cells, animal cells, plant cells, bacterial cells, germ-line cells, seed cells, sunflower cells, cotton cells, rapeseed cells, maize cells, palm cells, tobacco cells, peanut cells, soybean cells, *Arabidopsis sp.* cells, and *Ricinus sp.* cells.

Further aspects of the present invention provide plants comprising a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO:10. Examples of plants useful according to the present invention include, but are not limited to, sunflower, cotton, rapeseed, maize, palm, tobacco, peanut, soybean, *Arabidopsis sp.,* and *Ricinus sp.*

Further aspects of the present invention provide seeds comprising a nucleotide sequence of interest operatively linked to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10. Examples of seeds useful according to the present invention include, but are not limited to, seeds from sunflower, cotton, rapeseed, maize, palm, tobacco, peanut, soybean, *Arabidopsis sp.,* and *Ricinus sp.*

As will be apparent to one of ordinary skill in the art, a nucleotide sequence of interest may be any nucleotide sequence that one wishes to express. Examples of nucleotides sequences of interest include, but are not limited to, nucleotide sequences encoding proteins, ribozymes, antisense RNAs, siRNAs, RNAi molecules, markers, reporters, enzymes, signaling molecules, proteins involved in fatty acid synthesis, degradation, storage, and/or regulation, antisense RNAs targeted to RNAs encoding proteins involved in fatty acid synthesis, degradation, storage, and/or regulation, and siRNAs and/or RNAi molecules targeted to RNAs encoding proteins involved in fatty acid synthesis, storage, degradation, and/or regulation.

Further aspects of the present invention provide methods of expressing a nucleotide sequence of interest. One example of such a method comprises operatively linking an nucleotide sequence of interest to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10; and allowing the nucleotide sequence of interest to be expressed.

As will be apparent to one of ordinary skill in the art, allowing the nucleotide sequence of interest to be expressed relates generally to providing the operatively linked nucleotide sequences with or to an environment that allows for expression of the nucleotide sequence of interest. Examples of environments that allow for expression of the nucleotide sequence of interest are generally known in the art and include, but are not limited to, any environment having at least one dNTP and a polymerase, such as, but not limited to, an *in vitro* transcription kit, a PCR reaction, a cell, a eukaryotic cell, a prokaryotic cell, an animal cell, a plant cell, a bacterial cell, a germ-line cell, a seed cell, a sunflower cell, a cotton cell, a rapeseed cell, a maize cell, a palm cell, a tobacco cell, a peanut cell, a soybean cell, an *Arabidopsis sp.* cell, and a *Ricinus sp.* cell.

As will be appreciated by one of ordinary skill in the art, the operatively linked nucleotide sequences may be provided to an environment allowing expression using any procedure known in the art. Examples of such methods include, but are not limited to, transfection, using for example, lipofectin or lipofectamine, *Agrobacterium* mediated introduction (*see, e.g.,* Chistou (1996), Trends Plant Sci., 1: 423-432; and Hooykaas and Schilperoot (1992), Plant Mol. Bio., 19: 15-38), floral dip (*see, e.g.,* Clough and Bent (1998), Plant J., 16(6) 735-743), electroporation (*see, e.g.,* Shigekawa and Dower (1988), Biotechniques, 6:742; Miller, et al. (1988), Proc. Natl. Acad. Sci. USA, 85:856-860; and Powell, et al. (1988), Appl. Environ. Microbiol., 54:655-660); direct DNA uptake mechanisms (*see, e.g.,* Mandel and Higa (1972), J. Mol. Biol., 53:159-162; Dityatkin, et al. (1972), Biochimica et Biophysica Acta, 281:319-323; Wigler, et al. (1979), Cell, 16:77; and Uchimiya, et al. (1982), In: Proc. 5th Intl. Cong. Plant Tissue and Cell Culture, A. Fujiwara (ed.), Jap. Assoc. for Plant Tissue Culture, Tokyo, pp. 507- 508); fusion mechanisms (*see, e.g.,* Uchidaz, et al. (1980), In: Introduction of Macromolecules Into Viable Mammalian Cells, Baserga et al. (eds.) Wistar Symposium Series, 1:169-185); infectious agents (see Fraley, et al. (1986), CRC Crit. Rev. Plant Sci., 4:1-46); and Anderson (1984), Science, 226:401-409); microinjection mechanisms (*see, e.g.,* Crossway, et al. (1986), Mol. Gen. Genet., 202:179-185); and high velocity projectile mechanisms (*see, e.g.,* EPO 0 405 696 to Miller, Schuchardt, Skokut and Gould, (The Dow Chemical Company).

Particular embodiments of the present invention provide methods of expressing a nucleotide sequence of interest in a seed preferred manner. One example of such a method comprises: operatively linking a nucleotide sequence of interest to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO:10; directly or indirectly providing the operatively linked nucleotide sequences to a seed cell; and allowing for the expression of the nucleotide sequence of interest.

Other embodiments of the present invention provide methods of lowering the levels of a target protein. One example of such a method comprises: providing a nucleotide sequence encoding an antisense RNA and/or an siRNA that is capable of binding to an RNA encoding the target protein; operatively linking the nucleotide sequence encoding an antisense RNA and/or an siRNA to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10; and allowing the nucleotide sequence encoding an antisense RNA and/or an siRNA to be expressed.

Other aspects of the present invention provide methods of lowering the levels of a target protein in a seed preferred manner. One example of such a method comprises: providing a nucleotide sequence encoding an antisense RNA and/or an siRNA that is capable of binding to an RNA encoding the target protein; operatively linking the nucleotide sequence encoding an antisense RNA and/or an siRNA to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10; directly or indirectly providing the operatively linked nucleotide sequences to a seed cell; and allowing the nucleotide sequence encoding an antisense RNA and/or an siRNA to be expressed.

Further aspects of the present invention provide methods of changing the fatty acid content of a seed. One example of such a method comprises: operatively linking a nucleotide sequence encoding a protein involved in fatty acid synthesis, degradation, storage, and/or regulation to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO: 10; directly or indirectly providing the operatively linked nucleotide sequences to a seed cell; and allowing for the expression of the nucleotide sequence of interest. Examples of proteins involved in fatty acid synthesis, degradation, storage, and/or regulation include, but are not limited to, ACCase, FAS, KAS I, KAS II, KAS III, Fad2, and Fad3.

An additional example of a method of changing the fatty acid content of a seed comprises: providing a nucleotide sequence encoding an antisense RNA and/or an siRNA that is capable of binding to an RNA encoding a protein involved in fatty acid synthesis, degradation, storage, and/or regulation; operatively linking the nucleotide sequence encoding an antisense RNA and/or an siRNA to an isolated nucleotide sequence having at least about 60% identity, at least about 70% identity, at least about 80% identity, at least about 90% identity, or at least about 95% identity to a nucleotide sequence selected from SEQ ID NO:9 and SEQ ID NO:10; directly or indirectly providing the operatively linked nucleotide sequences to a seed cell; and allowing the nucleotide sequence encoding an antisense RNA and/or an siRNA to be expressed.

As used herein, a "promoter" refers to a region of DNA, generally upstream from the transcription initiation site, which is involved in recognition and binding of RNA polymerase and other proteins to initiate transcription. A promoter may include enhancers and repressors.

As used herein, "operably linked" refers to nucleotides sequences being joined so as to function in their intended manner. Sequences that are "operatively linked" may or may not be directly contiguous. For example, a promoter which is operatively linked to a nucleotide sequence of interest can be joined and positioned in such a way that it is capable of driving the expression of the nucleotide sequence of interest.

### EXAMPLES:

The present invention is further described in the following examples, which are offered by way of illustration and are not intended to limit the invention in any manner.

### Example 1

### Promoter Isolation

Genes encoding the legumin-like ("54-SSP") and oleosin proteins were identified *via* a random cDNA sequencing project, wherein gene annotation was accomplished using sequence-based homology to known genes. The promoters for these selected genes were isolated via PCR-based "genome walking," using a GenomeWalker™ kit (Clontech Laboratories, Inc.) according to the manufacturer's instructions. In this technique, short oligonucleotide adapter molecules with a known sequence were first ligated onto the ends of castor genomic DNA that had been digested with several different blunt-cutting restriction enzymes. A primary PCR reaction was carried out using a forward PCR primer that annealed to the adapter molecule and a reverse PCR primer that annealed specifically to the DNA within the coding region of the 54-SSP or oleosin gene. A second round of PCR using nested primers, also based on the known adapter and gene-specific sequences, was then carried out. Using this strategy, DNA sequences upstream from the selected coding sequences were isolated and inserted into the cloning vector pCR2.1 (Invitrogen Corp.) and sequenced. The primers used in this process are shown below.

### 54-SSP Primers

Primary PCR Reaction Primers
Adapter Primer 1 (forward primer): 5' GTA ATA CGA CTC ACT ATA GGG C 3' (SEQ ID NO:1)
54-SSP Primer B (reverse primer): 5' GAG AGC AGC GAA GAA GGC TGA ACC ATA G 3' (SEQ ID NO:2)

Nested PCR Reaction Primers:
Nested Adapter Primer 2 (forward primer): 5' ACT ATA GGG CAC GCG TGG T 3' (SEQ ID NO:3)
54-SSP Primer A (reverse primer): 5' GAG AGC CAT GGA AGA GAA CAA GTA GGA A 3' (SEQ ID NO:4)

### Oleosin Primers

Primary PCR Reaction Primers:
Adapter Primer 1 (forward primer): 5' GTA ATA CGA CTC ACT ATA GGG C3' (SEQ ID NO:5)
Oleosin Primer B (reverse primer): 5' ATA GGC TTG CAG AAT CAG AGC TTC TGG TTA 3' (SEQ ID NO:6)

Nested PCR Reaction Primers:
Nested Adapter Primer 2 (forward primer): 5' ACT ATA GGG CAC GCG TGG T 3' (SEQ ID NO:7)
Oleosin Primer A (reverse primer): 5' GGT GAC TAA CAA CCG GTG ATT GTT GAT GCT 3' (SEQ ID NO:8)

Once the sequences of the promoter fragments obtained in this manner were determined, it was possible to generate additional PCR products with specific cloning sites built into the primer sequences to facilitate expression vector construction.

The consensus nucleotide sequence of the 54-SSP promoter (1124 base pairs immediately preceding the 54-SSP start codon) can be found at SEQ ID NO:9.

The consensus nucleotide sequence of the oleosin promoter (528 base pairs immediately preceding the oleosin start codon) can be found at SEQ ID NO:10. It should be noted that the clone used for oleosin promoter testing had a PCR-generated sequence error, wherein the T at position 234 was replaced by an A.

### Example 2

### Testing of the promoters

The activities of the oleosin and 54-SSP gene promoters were tested in whole plants (*Arabidopsis thaliana*) through the use of the reporter gene Green Fluorescent Protein (GFP). Binary vectors for *Arabidopsis* transformation were constructed that had an expression cassette with the oleosin or 54-SSP promoter upstream of the CopGFP gene (Evrogen), followed by a Cauliflower Mosaic Virus 35S terminator region. Maps for the following vectors are shown in FIG. 1: pDOW2771 - 54-SSP promoter operatively linked to a CopGFP reporter gene; and pDOW2772 - Oleosin promoter operatively linked to a CopGFP reporter gene.

*Arabidopsis* plants were transformed by the floral dip method (Clough and Bent (1998) Plant Joumal 16:735-743). The binary vectors contained the phosphinothricin acetyltransferase (PAT) gene, which allowed selection of transformants on the herbicide glufosinate. In order to determine whether the promoters were active primarily in seeds, certain tissues were harvested from first generation (T1) transformants, including leaves, developing siliques, and mature seeds. These tissues were tested for CopGFP fluorescence as described below. The seeds from the T1 plants were planted to generate T2 plants, from which leaf, developing siliques, and mature seed samples were also taken and analyzed. Tissue samples were analyzed by the following CopGFP Assay procedure:
*CopGFP Assay:* Tissue samples were ground in 1X CCLR buffer (Cell Culture Lysis Buffer 5X Reagent (Promega Corp., catalog #E153A) containing 125 mM Tris (pH 7.8) with H₃PO₄, 10 mM CDTA, 10 mM DTT, 50% glycerol and 5% Triton X-100) for one minute in a tissue homogenizer. Samples were placed on dry ice until further use. The extracts were centrifuged at 14K x g for 15 minutes at 4°C. The supernatant fluid was transferred into another tube and used for further analysis. 200 µl of each supernatant was placed in a Costar 96-well flat bottom microtiter plate (Coming, Inc., catalog #3915) and fluorescence was measured using a SpectraMax Gemini XS microplate reader (Molecular Devices, Inc.). A culture containing an *E. coli* strain expressing CopGFP was used as a standard. The protein concentration of the tissue extracts was determined with a BCA protein assay kit (Pierce Biotechnology, Inc.; catalog #23225). Fluorescence was expressed as fluorescence per mg of protein. All experiments were performed in duplicate.

The results of these studies are presented in FIG. 2 and FIG. 3. For both the 54-SSP promoter and the oleosin promoter, there was much more activity in seeds than in vegetative leaf material. The developing siliques contain both vegetative tissue and developing seeds, and activities of the promoters in these tissues were between those of the leaf tissue and mature seeds.

Additional testing of the oleosin promoter was carried out in *Arabidopsis* plants using the luciferase gene as a reporter gene. The results of these experiments also indicated higher activity of the oleosin promoter in seeds.

### Example 3

### Vectors for the Modulation of Fatty Acid Synthesis Genes

Vectors for the modulation of fatty acid synthesis proteins are created as outlined in Example 2. The following vectors encoding fatty acid synthesis proteins under the control of the 54-SSP promoter are created:
pSSP:Fad2 - 54-SSP promoter operatively linked to a Fad2 gene;
pSSP:Fad3 - 54-SSP promoter operatively linked to a Fad3 gene; and
pSSP:KASII - 54-SSP promoter operatively linked to a KASII gene.

The following vectors encoding fatty acid synthesis proteins under the control of the Oleosin promoter are created:
pOleo:Fad2 - Oelosin promoter operatively linked to a Fad2 gene;
pOleo:Fad3 - Oelosin promoter operatively linked to a Fad3 gene; and
pOleo:KASII - Oelosin promoter operatively linked to a KASII gene.

The following vectors encoding antisense molecules against fatty acid synthesis proteins under the control of the 54-SSP promoter are created:
pSSP:Fad2:AS - 54-SSP promoter operatively linked to an antisense against Fad2 RNA;
pSSP:Fad3:AS - 54-SSP promoter operatively linked to an antisense against Fad3 RNA; and
pSSP:KASII:AS - 54-SSP promoter operatively linked to an antisense against KASII RNA.

The following vectors encoding antisense molecules against fatty acid synthesis proteins under the control of the Oleosin promoter are created:
pOleo:Fad2:AS - Oelosin promoter operatively linked to an antisense against Fad2 RNA;
pOleo:Fad3:AS - Oelosin promoter operatively linked to an antisense against Fad3 RNA; and
pOleo:KASII:AS - Oelosin promoter operatively linked to an antisense against KASII RNA.

Vectors encoding siRNA molecules against fatty acid synthesis proteins are created. The siRNA molecules may be designed to express an RNA molecule that hybridizes with itself to form a hairpin structure that comprises a single-stranded loop region and a base-paired stem. The base-paired stem region comprises a sense sequence corresponding to all or part of the endogenous messenger RNA encoding the gene whose expression is to be inhibited, and an antisense sequence that is fully or partially complementary to the sense sequence. Thus, the base-paired stem region of the molecule generally determines the specificity of the RNA interference. These hairpin RNA molecules are highly efficient at inhibiting the expression of endogenous genes, and the RNA interference they induce is inherited by subsequent generations. See, for example, Chuang and Meyerowitz (2000) Proc. Natl. Acad. Sci. USA 97:5985-5990; Stoutjesdijk et al. (2002) Plant Physiol. 129:1723-1731; and Waterhouse and Helliwell (2003) Nat. Rev. Genet. 5:29-38. Methods for using hpRNA interference to inhibit or silence the expression of genes are described, for example, in Chuang and Meyerowitz (2000) Proc. Natl. Acad. Sci. USA 97:5985- 5990; Stoutjesdijk et al. (2002) Plant Physiol. 129:1723-1731; Waterhouse and Helliwell (2003) Nat. Rev. Genet. 5:29-38; Pandolfini et al. BMC Biotechnology 3:7, and U.S. Patent Publication No. 20030175965. A transient assay for the efficiency of hairpin RNA constructs to silence gene expression in vivo has been described by Panstruga et al. (2003) Mol. Biol. Rep. 30:135-150*.*

The following vectors encoding siRNA molecules against fatty acid synthesis proteins under the control of the 54-SSP promoter are created:
pSSP:Fad2:si - 54-SSP promoter operatively linked to a siRNA against Fad2 RNA;
pSSP:Fad3:si - 54-SSP promoter operatively linked to a siRNA against Fad3 RNA; and
pSSP:KASII:si - 54-SSP promoter operatively linked to a siRNA against KASII RNA.

The following vectors encoding antisense molecules against fatty acid synthesis proteins under the control of the Oleosin promoter are created:
pOleo:Fad2:si - Oelosin promoter operatively linked to a siRNA against Fad2 RNA;
pOleo:Fad3:si - Oelosin promoter operatively linked to a siRNA against Fad3 RNA; and
pOleo:KASII:si - Oelosin promoter operatively linked to a siRNA against KASII RNA.

### Example 4

### Arabidopsis and Ricinus cultivation and transformation

*Arabidopsis* and *Ricinus* are cultivated under normal conditions. The vectors are introduced into Agrobacterium tumefaciens strain GV3101 pMP90 by electroporation and used to transform *Arabidopsis thaliana* and *Ricinus communis* plants by the floral dip method (N. Bechtold, J. Ellis, and G. Pelletier (1993) C. R. Acad. Sci. Paris 316, 1194-1198). Vectors are also introduced using high velocity particles and infectious agents Transformation is performed -5 days after initial flowering.

### Example 5

### Determination of Fatty Acid Content in Seeds of Arabidopsis and Ricinus

Fatty Acid Analysis: Seeds are methylated (1 ml of 1 N HCl, methanol, Supelco, 80°C for one hour), and extracted with hexane and trimethylsilylated (100 µl of BSTFA-TMCS (bis(treimethylsilyl)trifluoroacetamidetrimethylsilane), Supelco, 90°C for 45 minutes). The BSTFA-TMCS is removed by evaporation and the sample is resuspended in hexane. Samples are analyzed on a Hewlett-Packard 6890 gas chromatograph equipped with a 5973 mass selective detector (GC/MS) and a Supelco SP-2340 cyano capillary column (60 m x 250 µm x 0.25 µm). The injector is held at 225°C, the oven temperature is varied (100-240°C at 15°C/minute followed by 240°C for five minutes), and the helium flow is 1.1 ml/minute. Assignment of peak identities is performed based on elution time versus authentic standards and validated based on their mass spectra. Quantitation is performed using Hewlett-Packard chemstation software.

### Example 6

### Modulation of Fatty Acid Synthesis in Arabidopsis and Ricinus

Three methods of modulating fatty acid synthesis via Fad2 are compared (gene expression, antisense expression, siRNA expression). Three genes are chosen for the comparison of the three methods of gene repression (the 12-desatuarse FAD2 and the 15-desaturase FAD3 because they are easily scored; and the FAD2 because it had been used before for evaluation of reduction in gene expression), as well as β-ketoacyl-ACP synthase (KAS) II. The relationship between these enzymes and fatty acid synthesis is depicted in FIG. 4.

Seeds are chosen for analysis because they allow for reproducible quantitative analysis of their composition by gas chromatography and because they allow for mass spectrometric analysis of peaks to qualitatively confirm the assignments of peaks as specific fatty acids. Student T-test is used to assign significance to differences between means (based on ten or more samples per mean).

### Example 7

### Modulation of KASII Expression

KASII elongates 16 C to 18 C fatty acids in the plastid. For KASII, levels of 16:0 plus 16:1 fatty acids (the substrates for KASII) are compared with the levels of its product 18:0 and 18:1 plus metabolites 18:2 and 18:3.

Wild-type *Arabidopsis* and *Ricinus* are compared to lines transformed with pSSP:KASII, pOleo:KASII, pSSP:KASII:AS, pOleo:KASII:AS, pSSP:KASII:si, or pOleo:KASII:si. Those plants harboring the pSSP:KASII and pOleo:KASII vectors show significant decreases in 16:0 fatty acids with corresponding increases in C18 and higher fatty acids. Those plants harboring the pSSP:KASII:AS, pOleo:KASII:AS, pSSP:KASII:si, and pOleo:KASII:si vectors show significant increases in 16:0 fatty acids with corresponding decreases in C18 and higher fatty acids.

### Example 8

### Modulation of FAD2 Expression

For FAD2, levels of 18:1 fatty acids (the substrate for FAD2) are compared with the levels of its product 18:2 and the metabolite 18:3. For analysis, 18:2 is summed with 18:3 to get the total fatty acid proportion that had been desaturated by FAD2.

Wild-type *Arabidopsis* and *Ricinus* are compared to lines transformed with pSSP:FAD2, pOleo:FAD2, pSSP:FAD2:AS, pOleo:FAD2:AS, pSSP:FAD2:si, or pOleo:FAD2:si. Those plants harboring the pSSP:FAD2 and pOleo:FAD2 vectors show significant increases in 18:2 and 18:3 fatty acids. Those plants harboring the pSSP:FAD2:AS, pOleo:FAD2:AS, pSSP:FAD2:si, and pOleo:FAD2:si vectors show significant decreases in 18:2 and 18:3 fatty acids with corresponding increases in lower order fatty acids.

### Example 9

### Modulation of FAD3 Expression

For FAD3, levels of 18:1 plus 18:2 fatty acids (18:2 being the substrate for FAD3) are compared with the levels of its product 18:3.

Wild-type *Arabidopsis* and *Ricinus* are compared to lines transformed with pSSP:FAD3, pOleo:FAD3, pSSP:FAD3:AS, pOleo:FAD3:AS, pSSP:FAD3:si, or pOleo:FAD3:si. Those plants harboring the pSSP:FAD3 and pOleo:FAD3 vectors show significant increases in 18:3 fatty acids. Those plants harboring the pSSP:FAD3:AS, pOleo:FAD3:AS, pSSP:FAD3:si, and pOleo:FAD3:si vectors show significant decreases in 18:3 fatty acids with corresponding increases in lower order fatty acids.

While this invention has been described in certain example embodiments, the present invention may be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

The present application and invention further includes the subject matter of the following numbered clauses:
1. An isolated nucleotide sequence comprising a nucleotide sequence having at least about 90% homology to the sequence of SEQ ID NO:9 or SEQ ID NO:10.
2. The isolated nucleotide sequence of clause 1, further comprising a nucleotide sequence of interest operatively linked to the nucleotide sequence having at least about 90% homology to the sequence of SEQ ID NO:9 or SEQ ID NO:10.
3. The isolated nucleotide sequence of clause 2, wherein the nucleotide sequence of interest encodes a molecule selected from the group consisting of proteins, antisense oligonucleotides, and siRNAs.
4. A vector comprising the isolated nucleotide sequence of clause 1,
5. The vector according to clause 4, further comprising a nucleotide sequence of interest operatively linked to the nucleotide sequence having at least about 90% homology to the sequence of SEQ ID NO:9 or SEQ ID NO:10.
6. The vector of clause 4, wherein the nucleotide sequence encodes a molecule selected form the group consisting of proteins, antisense oligonucleotides, and siRNAs.
7. The vector according to clause 5, further comprising a selectable marker.
8. The vector according to clause 7, wherein the selectable marker is a phosphinothricin acetyltransferase gene.
9. A cell comprising the isolated nucleotide sequence of clause 1.
10. The cell of clause 9, wherein the isolated nucleotide sequence of clause 1 is stably integrated in the genome of the cell.
11. The cell according to clause 9, wherein the cell is selected from the group consisting of eukaryotic, prokaryotic, animal, plant, germ-line, seed, *Arabidopsis sp.,* sunflower, cotton cells, rapeseed, maize, palm, tobacco, peanut, soybean, and *Ricinus sp.* cells.
12. The cell of clause 9, wherein the cell comprises the vector of clause 4.
13. A plant comprising the isolated nucleotide sequence of clause 1.
14. The plant according to clause 13, wherein the plant is selected from the group consisting of *Arabidopsis sp.,* sunflower, cotton cells, rapeseed, maize, palm, tobacco, peanut, soybean, and *Ricinus sp.*
15. The plant according to clause 13, wherein the plant comprises the vector of clause 4.
16. The plant according to clause 13, wherein the plant comprises the cell of clause 9.
17. A seed comprising the isolated nucleotide sequence of clause 1.
18. The seed according to clause 17, wherein the seed is selected from the group consisting of *Arabidopsis sp.,* sunflower, cotton cells, rapeseed, maize, palm, tobacco, peanut, soybean, and *Ricinus sp.* seeds.
19. The seed according to clause 17, wherein the plant comprises the vector of clause 4.
20. The seed according to clause 17, wherein the plant comprises the cell of clause 9.
21. A method of promoting the expression of a molecule encoded by a nucleotide sequence of interest, the method comprising: operatively linking the isolated nucleotide sequence of clause 1 to the nucleotide sequence of interest.
22. The method according to clause 21, wherein the expression of the molecule encoded by the nucleotide sequence of interest is promoted in a seed preferred manner.
23. The method of clause 21, wherein the molecule encoded by the nucleotide sequence of interest is selected from the group consisting of a proteins, antisense oligonucleotides, and siRNAs.
24. A method of producing a molecule encoded by a nucleotide sequence of interest in a cell, the method comprising:
   operatively linking the isolated nucleotide sequence of clause 1 to the nucleotide sequence of interest; and
   providing the operatively linked nucleotide sequences to the cell;
   expressing the nucleotide sequence of interest.
25. The method of clause 24, wherein the molecule encoded by the nucleotide sequence of interest is selected from the group consisting of proteins, antisense oligonucleotides, and siRNAs.
26. A method of modulating the expression of a target in a cell, the method comprising:
   providing a nucleotide sequence of interest, wherein the nucleotide sequence encodes an antisense oligonucleotide or an siRNA that is capable of modulating the expression of the target in a cell;
   operatively linking the isolated nucleotide sequence of clause 1 to the nucleotide sequence of interest;
   providing the operatively linked nucleotide sequences to the cell; and
   expressing the nucleotide sequence of interest.
27. The method according to clause 26, wherein the cell is selected form the group consisting of prokaryotic, eukaryotic, bacterial, agrobactrerium, yeast, plant, mammalian, and human cells.
28. The method according to clause 26, herein the plant cell is selected form the group consisting of *Ricinus, Arabidopsis,* sunflower, cotton, rapeseed, maize, palm, tobacco, peanut or soybean cells.
29. The method according to clause 26, wherein the target is a gene, oligonucleotide sequence, and/or a protein.
30. The method according to clause 26, wherein the target is selected from proteins involved in fatty acid synthesis, degradation, storage, and/or regulation
31. The method according to clause 26, wherein the target is selected from the group consisting of ACCase, FAS, KAS I, KAS II, KAS III, Fad2, and Fad3.
32. A genetic descendant of the plant of clause 13.

## Claims

1. An isolated nucleotide sequence comprising a nucleotide sequence having at least 90% identity to the sequence of SEQ ID NO:10, wherein the isolated nucleotide sequence is capable of acting as a promoter.

2. A vector comprising the isolated nucleotide sequence of claim 1.

3. The isolated nucleotide according to claim 1 or the vector according to claim 2, further comprising a nucleotide sequence of interest operatively linked to the nucleotide sequence having at least 90% identity to the sequence of SEQ ID NO:10.

4. The isolated nucleotide or the vector according to claim 3, wherein the nucleotide sequence of interest encodes a molecule selected form the group consisting of proteins, antisense oligonucleotides, and siRNAs.

5. The vector according to claim 3, further comprising a phosphinothricin acetyltransferase gene as a selectable marker.

6. A cell comprising as a transgene the isolated nucleotide sequence of claim 1.

7. A plant comprising as a transgene the isolated nucleotide sequence of claim 1.

8. A seed comprising as a transgene the isolated nucleotide sequence of claim 1.

9. The cell according to claim 6, the plant according to claim 7, or the seed according to claim 8, wherein the cell, plant, or seed is selected from the group consisting of *Arabidopsis sp.,* sunflower, cotton cells, rapeseed, maize, palm, tobacco, peanut, soybean, and *Ricinus sp.*

10. A method of promoting the expression of a molecule encoded by a nucleotide sequence of interest, the method comprising: operatively linking the isolated nucleotide sequence of claim 1 to the nucleotide sequence of interest.

11. The method according to claim 10, wherein the expression of the molecule encoded by the nucleotide sequence of interest is promoted in a seed preferred manner.

12. A method of producing a molecule encoded by a nucleotide sequence of interest in a cell, the method comprising: operatively linking the isolated nucleotide sequence of claim 1 to the nucleotide sequence of interest; and providing the operatively linked nucleotide sequences to the cell; expressing the nucleotide sequence of interest.

13. The method of claim 10 or claim 12, wherein the molecule encoded by the nucleotide sequence of interest is selected from the group consisting of a proteins, antisense oligonucleotides and siRNAs.

14. A method of modulating the expression of a target in a cell, the method comprising:
providing a nucleotide sequence of interest, wherein the nucleotide sequence encodes an antisense oligonucleotide or an siRNA that is capable of modulating the expression of the target in a cell; operatively linking the isolated nucleotide sequence of claim 1 to the nucleotide sequence of interest; providing the operatively linked nucleotide sequences to the cell; and
expressing the nucleotide sequence of interest.

15. The method according to claim 14, wherein the cell is a plant cell and is selected form the group consisting of *Ricinus, Arabidopsis,* sunflower, cotton, rapeseed, maize, palm, tobacco, peanut or soybean cells.

16. The method according to claim 14, wherein the target is selected from the group consisting of ACCase, FAS, KAS I, KAS II, KAS III, Fad2, and Fad3.
